# EUROPEAN PATENT SPECIFICATION

(11) **EP 1 546 302 B1**
(45) Date of publication and mention of the grant of the patent: **12.08.2009**
(21) Application number: 03755759.2
(22) Date of filing: 29.08.2003
(51) Int. Cl.: C12N 9/76, C12N 1/15, C12N 15/52, C12N 15/62, C12P 21/02

(54) **METHODS FOR PRODUCING MAMMALIAN TRYPSINS**
VERFAHREN ZUR HERSTELLUNG VON SÄUGETIER-TRYPSINEN
PROCEDE DE FABRICATION DE TRYPSINES DE MAMMIFERE

(30) Priority: 30.08.2002 US 407170 P
(43) Date of publication of application: 29.06.2005
(73) Proprietor: Novozymes, Inc., Davis, CA 95616 (US)
(72) Inventor: BERKA, Randy, Davis, CA 95616 (US); BROWN, Kimberly, Elk Grove, CA 95758 (US)
(74) Representative: Hansen, Rasmus Rune
(86) International application number: PCT/US2003/027074
(87) International publication number: WO 2004/020612

(56) References cited:
- WO-A-96/00787
- WO-A-02/061064
- WO-A1-00/17332
- WO-A2-97/26330
- US-A- 5 945 328
- US-B1- 6 180 366

## Description

### Background of the Invention

### Field of the invention

The present invention relates to methods for producing mammalian trypsins in *Fusarium venenatum* host strains.

### Description of the Related Art

Proteolytic enzymes have widespread commercial application and have been successfully implemented in different industries such as the detergent, leather, chemical, agricultural, pharmaceutical, food, and dairy industries.

Trypsin is a proteolytic enzyme available from mammalian sources and is useful commercially. For example, pancreatic trypsin, derived from porcine pancreas, is used in the food functionality industry and for neutral and alkaline bating of hides and skins in the leather industry.

Trypsin preferentially cleaves at the C-terminal side of peptide bonds of the L-isomers of lysine and arginine. Trypsin is synthesized as a precursor known as trypsinogen having both an amino-terminal signal peptide to direct secretion as well as a propeptide that silences enzyme activity until it is proteolytically removed with concomitant activation of the enzyme. Cleavage of the propeptide requires a highly specific serine endoprotease activity termed enterokinase (enteropeptidase) which activates trypsinogen by cleavage following the sequence (Asp)₄-Lys (Lavallie et al., 1993, Journal of Biological Chemistry 268: 2331-23317).

WO 02/061064 discloses the recombinant expression of porcine trypsinogen in Pichia pastoris, whereby the trypsinogen signal sequence and propeptide sequence have been deleted except for the enterokinase recognition sequence. Trypsinogen is then autocatalytically activated into trypsin. WO 96/00787 discloses the use of Fusarium graminearum (Fusarium venenatum) ATCC 20334 as host cell for the production of recombinant proteins, in particular the trypsin-like protease of Fusarium oxysporum. U.S. Patent No. 5,945,328 discloses the expression of a porcine trypsin in an *Aspergillus oryzae* host strain, by transforming the fungus with a vector comprising the trypsinogen DNA N-terminally fused to a signal sequence, such as the native trypsinogen signal. WO 01/55429 discloses a method for the manufacture and purification of recombinant human trypsinogen and trypsin in *E*. coli and yeast. WO 00/17332 discloses trypsin and trypsinogen analogs, produced recombinantly in *E*. *coli and Pichia pastoris,* and nucleic acids thereof, which contain modifications of the trypsinogen leader sequence such that the trypsinogen cannot be cleaved by trypsin or trypsin-like enzymes. WO 99/10503 discloses a process in *E*. *coli* for the recombinant production of trypsinogen as inclusion bodies where the trypsinogen contains an autocatalytic cleavage site which does not occur naturally, but is recognized by the active form of the enzyme trypsin. However, the expression of mammalian trypsins in microbial systems has not been achieved in commercially relevant levels and, thus, there is a need in the art to provide more suitable expression systems in microorganisms for the production of commercial quantities of mammalian trypsin.

The object of the present invention is to provide methods for producing a mammalian trypsin in a *Fusarium venenatum* host.

### Summary of the Invention

The present invention relates to methods for producing a mammalian trypsin, the method comprising:
(a) cultivating a *Fusarium venenatum* host strain in a culture medium under conditions suitable for expression of the mammalian trypsin and secretion thereof into the medium, wherein the *Fusarium venenatum* host strain comprises a nucleic acid construct comprising a nucleic acid sequence encoding the mature coding sequence of the mammalian trypsin operably linked to nucleotides 58 to 129 of SEQ ID NO: 1 encoding the signal peptide and propeptide of *Fusarium oxysporum* trypsinogen; and
(b) recovering the mammalian trypsin from the medium.

The present invention also relates to constructs comprising a nucleic acid sequence encoding the mature coding sequence of a mammalian trypsin operably linked to nucleotides 58 to 129 of SEQ ID NO: 1 encoding the signal peptide and propeptide of *Fusarium oxysporum* trypsinogen, and to vectors and *Fusarium venenatum* host strains comprising such constructs.

### Brief Description of the Figures

Figures 1A and 1B show the genomic DNA sequence and the deduced amino acid sequence of a *Fusarium oxysporum* trypsinogen-like protein (SEQ ID NOS: 1 and 2, respectively).
Figures 2A, 2B, and 2C show the cDNA sequence and the deduced amino acid sequence of a porcine trypsinogen (SEQ ID NOS: 3 and 4, respectively).
Figure 3 shows a restriction map of pCaHj522.
Figure 4 shows a restriction map of pRaMB58.

### Detailed Description of the Invention

The present invention relates to methods for producing a mammalian trypsin in a *Fusarium venenatum* host strain, the method comprising (a) cultivating the *Fusarium venenatum* host strain in a culture medium under conditions suitable for.expression of the mammalian trypsin and secretion thereof into the medium, wherein the *Fusarium venenatum* host strain comprises a nucleic acid construct comprising a nucleic acid sequence encoding the mature coding sequence of the mammalian trypsin operably linked to nucleotides 58 to 129 of SEQ ID NO: 1 encoding the signal peptide and propeptide of *Fusarium oxysporum* trypsinogen; and (b) recovering the mammalian trypsin from the medium.

The present invention advantageously provides methods for producing mammalian trypsins, particularly porcine trypsin, in a filamentous fungal host strain where the prepro form of the enzyme is correctly processed to mature active trypsin.

The term "trypsin" is defined herein as an endopeptidase which catalyzes the hydrolysis of carboxylic acid amides with preferential cleavage at the C-terminal side of the L-isomer of Arg or Lys (E.C. 3.4.21.4). In the present invention, the term "trypsin" will be understood to mean the mature enzyme, *i.e*., an active trypsin without an amino-terminal signal peptide and a propeptide. Trypsins are biosynthesized as precursors having both an amino-terminal signal peptide to direct secretion as well as a propeptide that silences enzyme activity until it is proteolytically removed with concomitant activation of the enzyme. The precursor form of the enzyme is known as trypsinogen.

For purposes of the present invention, trypsin activity is determined using N-alpha-benzoyl-L-arginine p-nitroanilide hydrochloride as substrate according to the procedure Gaertner and Puigserver, 1992, Enzyme Microb. Technol. 14: 150, at 25°C with 2 mg of N-alpha-benzoyl-L-arginine p-nitroanilide hydrochloride per ml of 100 mM MOPS buffer, 4 mM CaCl₂, 0.01% Triton X-100, pH 7.5. The activity is monitored at 405 nm. One unit of trypsin activity is defined as 1.0 µmole of N-alpha-benzoyl-L-arginine p-nitroanilide hydrolyzed per minute at 25°C, pH 6.5.

### Mammalian Trypsins/Trypsinogens

In the present invention, a nucleic acid sequence encoding a mammalian trypsin or trypsinogen may be obtained from any mammalian source. The term "obtained from" as used herein in connection with a given source means that the trypsin encoded by a nucleic acid sequence is produced by the source or by a cell in which the nucleic acid sequence from the source has been inserted.

The techniques used to isolate or clone a nucleic acid sequence encoding a mammalian trypsin or trypsinogen are known in the art and include isolation from genomic DNA, preparation from cDNA, or a combination thereof. A variety of techniques are available including, for example, hybridization, polymerase chain reaction (PCR) amplification, or *de novo* DNA synthesis. The nucleic acid sequence may be of genomic, cDNA, RNA, semisynthetic, synthetic origin, or any combinations thereof.

The cloning procedures may involve excision and isolation of a desired nucleic acid fragment comprising the nucleic acid sequence encoding the mammalian trypsin or trypsinogen, insertion of the fragment into a vector molecule, and incorporation of the recombinant vector into a *Fusarium venenatum* host strain where multiple copies or clones of the nucleic acid sequence will be replicated.

Oligonucleotide primers targeted to any suitable region of a trypsinogen gene can be used for PCR amplification of the gene. See, for example, Innis et al., 1990, PCR Protocols: A Guide to Methods and Application, Academic Press, New York. The PCR amplification comprises template DNA, suitable enzymes, primers, and buffers, and is conveniently carried out in a DNA thermal cycler.

cDNA can be isolated from a library constructed from any mammalian tissue in which a trypsinogen gene is expressed. Methods for constructing cDNA libraries in a suitable vector such as a plasmid or phage for propagation in prokaryotic or eukaryotic cells are well known in the art. (See, for example, Sambrook, 1989, *supra).* For example, mRNA is isolated from a suitable tissue, and first strand cDNA synthesis is carried out. A second round of DNA synthesis can be carried out for the production of the second strand. The double-stranded cDNA can be cloned into any suitable vector, for example, a plasmid, thereby forming a cDNA library.

The isolated nucleic acid sequences can also be prepared by direct chemical synthesis by methods such as the phosphotriester method of Narang et al., 1979, Methods of Enzymology 68:90-99; the phosphodiester method of Brown et al., 1979, Methods of Enzymology 68: 109-151; the diethylphosphoramidite method of Beaucage et al., 1981, Tetrahedron Letters 22: 1859-1862; and the solid phase phosphoramidetriester method of Beaucage and Carothers, 1981, Tetrahedron Letters. 22: 1859-1862. Chemical synthesis generally produces a single-stranded oligonucleotide, which may be converted into double-stranded DNA by hybridization with a complementary sequence, or by polymerization with a DNA polymerase using the single strand as a template.

In a preferred embodiment, the nucleic acid sequence encoding a mammalian trypsin or trypsinogen may be, but is not limited to, one of the following:

| Trypsin/Trypsinogen | Accession No. |
|---|---|
| Bovine pancreatic trypsinogen | Genbank X54703 |
| Cow pancreatic trypsinogen | Genbank AF453325 |
| Dog pancreatic trypsinogen | Genbank M11589, M11590 |
| Human trypsinogen 1 | Swissprot P07477 |
| Human pancreatic trypsin 1 | Genbank M22612 |
| Human pancreatic trypsin II | Genbank M27602 |
| Human pancreatic trypsinogen III | Genbank X15505 |
| Human pancreatic trypsinogen IVa | Genbank X72781 |
| Human pancreatic trypsinogen IVb | Genbank X71345 |
| Mouse pancreatic trypsin | Genbank AB017030, AB017032 |
| Pig trypsinogen | Geneseqn AAT49878 |
| Rat pancreatic trypsinogen I | Genbank V01273 |
| Rat pancreatic trypsinogen II | Genbank V01274 |
| Rat pancreatic trypsin | Genbank J00778 |
| Rat trypsin Va | Genbank X59012 |
| Rat trypsin Vb | Genbank X59013 |

In a more preferred embodiment, the nucleic acid sequence encoding a mammalian trypsin or trypsinogen is pig (porcine) trypsinogen or trypsin. In a most preferred embodiment, the nucleic acid sequence encoding a mammalian trypsin or trypsinogen is the pig trypsinogen of Geneseqn AAT49878.

### Fusarium venenatum Host Strains

In the methods of the present invention, any *Fusarium venenatum* strain may be used as a host strain for producing a mammalian trypsin.

In a preferred embodiment, the *Fusarium venenatum* host strain is *Fusarium venenatum* A3/5, which was originally deposited as *Fusarium graminearum* ATCC 20334 and recently reclassified as *Fusarium venenatum* by Yoder and Christianson, 1998, Fungal Genetics and Biology 23: 62-80 and O'Donnell et al., 1998, Fungal Genetics and Biology 23: 57-67; as well as taxonomic equivalents of *Fusarium venenatum* regardless of the species name by which they are currently known.

In another preferred embodiment, the *Fusarium venenatum* host strain is a morphological mutant of *Fusarium venenatum* A3/5 or *Fusarium venenatum* ATCC 20334, as disclosed in WO 97/26330.

In another preferred embodiment, the *Fusarium venenatum* host strain is a trichothecene-deficient *Fusarium venenatum* strain. See, for example, U.S. Patent No. 6,180,366.

In a further preferred embodiment, the *Fusarium venenatum* host strain is a cyclohexadepsipeptide-deficient *Fusarium venenatum* strain. See, for example, WO 00/42203.

### Nucleic Acid Constructs

"Nucleic acid construct" is defined herein as a nucleic acid molecule, either single- or double-stranded, which is isolated from a naturally occurring gene or which has been modified to contain segments of nucleic acid which are combined and juxtaposed in a manner which would not otherwise exist in nature. The term nucleic acid construct is synonymous with the term expression cassette when the nucleic acid construct contains all the control sequences required for expression of a coding sequence. The term "coding sequence" as defined herein is a sequence which is transcribed into mRNA and translated into a polypeptide. The boundaries of the genomic coding sequence are generally determined by the ATG start codon located at the beginning of the open reading frame at the 5' end of the mRNA to the stop codon (TAA, TAG, or TGA) followed by a transcription terminator sequence located just downstream of the open reading frame at the 3' end of the mRNA. A coding sequence can include, but is not limited to, genomic, cDNA, RNA, semisynthetic, synthetic, recombinant, or any combinations thereof.

In the methods of the present invention, the nucleic acid constructs comprise a nucleic acid sequence encoding the mature coding sequence of an active mammalian trypsin operably linked to nucleotides 58 to 129 of SEQ ID NO: 1 encoding the signal peptide (nucleotides 58 to 111) and propeptide (nucleotides 112 to 129) of *Fusarium oxysporum* trypsinogen. The sequence of SEQ ID NO: 1 is obtainable from *Fusarium oxysporum* DSM 2672 (U.S. Patent No. 5,693,520).

The propeptide region is positioned next to the amino terminus of mature coding sequence of the mammalian trypsin and the signal peptide region is positioned next to the amino terminus of the propeptide region.

The propeptide coding region (nucleotides 112 to 129) codes for an amino acid sequence (amino acids 18 to 24 of SEQ ID NO: 2) linked in translation reading frame with the amino terminus of the mature mammalian trypsin. The resultant polypeptide is known as a proenzyme or propolypeptide. A propolypeptide is generally inactive and can be converted to a mature, active polypeptide by catalytic or autocatalytic cleavage of the propeptide from the propolypeptide. In the present invention, the *Fusarium oxysporum* propeptide coding region replaces the native propeptide coding region of the mammalian trypsin to achieve proper processing of the trypsin precursor by endogenous protease activity in the *Fusarium venenatum* host strain.

The signal peptide coding region (nucleotides 58 to 129 of SEQ ID NO: 1) encodes an amino acid sequence (amino acids 1 to 17 of SEQ ID NO: 2) linked in translation reading frame with the amino terminus of the propeptide coding region and directs the trypsinogen into the microorganism's secretory pathway. In the present invention, the *Fusarium oxysporum* signal peptide coding region replaces the native signal peptide coding region of the mammalian trypsinogen to direct the molecule into the secretory pathway of the *Fusarium venenatum* host strain.

An isolated nucleic acid sequence encoding a mammalian trypsin may be further manipulated in a variety of ways to provide for expression of the mammalian trypsin in a *Fusarium venenatum* host strain. Expression will be understood to include any step involved in the production of the mammalian trypsin including, but not limited to, transcription, post-transcriptional modification, translation, post-translational modification, and secretion. Manipulation of the nucleic acid sequence prior to its insertion into a vector may be desirable or necessary depending on the expression vector. The techniques for modifying nucleic acid sequences utilizing cloning methods are well known in the art.

The term "control sequences" is defined herein to include all components which are necessary or advantageous for the expression of a mammalian trypsin. Each control sequence may be native or foreign to the nucleic acid sequence encoding the mammalian trypsin. Besides the propeptide sequence and signal sequence described above, the control sequences also include, but are not limited to, a leader, a polyadenylation sequence, a promoter, and a transcription terminator. At a minimum, the control sequences include a promoter, and transcriptional and translational start and stop signals. The control sequences may be provided with linkers for the purpose of introducing specific restriction sites facilitating ligation of the control sequences with the coding region of the nucleic acid sequence encoding a mammalian trypsin. The term "operably linked" is defined herein as a configuration in which a control sequence is appropriately placed at a position relative to the coding sequence of the DNA sequence such that the control sequence directs the production of a mammalian trypsin.

The control sequence may be an appropriate promoter sequence, a nucleic acid sequence which is recognized by a *Fusarium venenatum* host strain for expression of the nucleic acid sequence encoding mammalian trypsin. The promoter sequence contains transcriptional control sequences which mediate the expression of the mammalian trypsin. The promoter may be any nucleic acid sequence which shows transcriptional activity in the *Fusarium venenatum* host strain including mutant, truncated, and hybrid promoters, and may be obtained from genes encoding extracellular or intracellular polypeptides either homologous or heterologous to the cell. The promoter may be preceded by activating sequences and enhancer sequences known in the art.

Examples of suitable promoters for directing the transcription of the nucleic acid constructs in the methods of the present invention are promoters obtained from the genes encoding *Aspergillus* oryzae TAKA amylase, *Rhizomucor miehei* aspartic proteinase, *Aspergillus niger* neutral alpha-amylase, *Aspergillus niger* acid stable alpha-amylase, *Aspergillus niger* or *Aspergillus awamori* glucoamylase *(glaA), Rhizomucor miehei* lipase, *Aspergillus oryzae* alkaline protease, *Aspergillus oryzae* triose phosphate isomerase, *Aspergillus nidulans* acetamidase, *Aspergillus oryzae* acetamidase, *Fusarium oxysporum* trypsin-like protease, *Fusarium venenatum* glucoamylase promoter, *Fusarium venenatum* Daria promoter, *Fusarium venenatum* Quinn promoter, and mutant, truncated, and hybrid promoters thereof, as well as NA2-tpi promoters (a hybrid of the promoters from the genes encoding *Aspergillus niger* neutral alpha-amylase and *Aspergillus oryzae* triose phosphate isomerase). In a preferred embodiment, the promoter is obtained from the *Fusarium oxysporum* trypsin-like gene promoter, *Fusarium venenatum* glucoamylase gene promoter, *Fusarium venenatum* Daria gene promoter, *Fusarium venenatum* Quinn gene promoter.

The control sequence may also be a suitable transcription terminator sequence, a sequence recognized by a *Fusarium venenatum* host strain to terminate transcription. The terminator sequence is operably linked to the 3' terminus of the nucleic acid sequence encoding the mammalian trypsin. Any terminator which is functional in the *Fusarium venenatum* host strain may be used in the present invention.

In a preferred embodiment, the terminator is obtained from the genes encoding *Aspergillus oryzae* TAKA amylase, *Aspergillus niger* glucoamylase, *Aspergillus nidulans* anthranilate synthase, *Aspergillus niger* alpha-glucosidase, and *Fusarium oxysporum* trypsin-like protease.

The control sequence may also be a suitable leader sequence, a nontranslated region of a mRNA which is important for translation by the *Fusarium venenatum* host strain. The leader sequence is operably linked to the 5' terminus of the nucleic acid sequence encoding the mammalian trypsin. Any leader sequence which is functional in the *Fusarium venenatum* host strain may be used in the present invention.

Preferred leaders are obtained from the genes encoding *Aspergillus oryzae* TAKA amylase and *Aspergillus nidulans* triose phosphate isomerase.

The control sequence may also be a polyadenylation sequence, a sequence which is operably linked to the 3' terminus of the nucleic acid sequence and which, when transcribed, is recognized by a *Fusarium venenatum* host strain as a signal to add polyadenosine residues to transcribed mRNA. Any polyadenylation sequence which is functional in the *Fusarium venenatum* host strain may be used in the present invention.

Preferred polyadenylation sequences are obtained from the genes encoding *Aspergillus oryzae* TAKA amylase, *Aspergillus niger* glucoamylase, *Aspergillus nidulans* anthranilate synthase, and *Aspergillus niger* alpha-glucosidase.

The nucleic acid constructs may also comprise one or more nucleic acid sequences which encode one or more factors that are advantageous for directing the expression of the mammalian trypsin, *e.g.,* a transcriptional activator (*e.g.,* a trans-acting factor), a chaperone, and a processing protease. Any factor that is functional in a *Fusarium venenatum* host strain may be used in the present invention. The nucleic acids encoding one or more of these factors are not necessarily in tandem with the nucleic acid sequence encoding the mammalian trypsin.

It may also be desirable to add regulatory sequences which allow regulation of expression of the mammalian trypsin relative to the growth of the *Fusarium venenatum* host strain. Examples of regulatory systems are those which cause the expression of the gene to be turned on or off in response to a chemical or physical stimulus, including the presence of a regulatory compound. The TAKA alpha-amylase promoter, *Aspergillus niger* glucoamylase promoter, and *Aspergillus oryzae* glucoamylase promoter may be used as regulatory sequences. Other examples of regulatory sequences are those which allow for gene amplification, e.g., the metallothionein genes which are amplified with heavy metals. In these cases, the nucleic acid sequence encoding the mammalian trypsin would be operably linked with the regulatory sequence.

### Recombinant Expression Vectors

The various nucleic acid and control sequences described above may be joined together to produce a recombinant expression vector which may include one or more convenient restriction sites to allow for insertion or substitution of the nucleic acid sequence encoding the mammalian trypsin at such sites. Alternatively, the nucleic acid sequence encoding the mammalian trypsin may be expressed by inserting the sequence or a nucleic acid construct comprising the sequence into an appropriate vector for expression. In creating the expression vector, the coding sequence is located in the vector so that the coding sequence is operably linked with the appropriate control sequences for expression and secretion.

The recombinant expression vector may be any DNA or RNA molecule (e.g., a plasmid, linear fragment, or virus) which can be conveniently subjected to recombinant DNA procedures and can bring about the expression of the nucleic acid sequence encoding the mammalian trypsin in a *Fusarium venenatum* host strain. The choice of the vector will typically depend on the compatibility of the vector with the *Fusarium venenatum* host strain into which the vector is to be introduced. The vectors may be linear or closed circular plasmids. The vector may be an autonomously replicating vector, i.e., a vector which exists as an extrachromosomal entity, the replication of which is independent of chromosomal replication, e.g., a plasmid, an extrachromosomal element, a minichromosome, or an artificial chromosome. The vector may contain any means for assuring self-replication. Alternatively, the vector may be one which, when introduced into the *Fusarium venenatum* host strain, is integrated into the genome and replicated together with the chromosome(s) into which it has been integrated. The vector system may be a single vector or plasmid or two or more vectors or plasmids which together contain the total DNA to be introduced into the genome of the *Fusarium venenatum* host strain, or a transposon.

The vectors may contain one or more selectable markers which permit easy selection of transformed *Fusarium venenatum* host cells. A selectable marker is a gene the product of which provides for biocide or viral resistance, resistance to heavy metals, prototrophy to auxotrophs, and the like. A selectable marker for use in a *Fusarium venenatum* host strain may be selected from the group including, but not limited to, *amdS* (acetamidase), *argB* (ornithine carbamoyltransferase), bar (phosphinothricin acetyltransferase), *hph* (hygromycin phosphotransferase), *niaD* (nitrate reductase), *pyrG* (orotidine-5'-phosphate decarboxylase), sC (sulfate adenyltransferase), and *trpC* (anthranilate synthase), as well as equivalents from other species. Preferred for use in a *Fusarium venenatum* host strain are the *amdS* and *pyrG* genes of *Aspergillus nidulans* or *Aspergillus oryzae,* the *niaD* gene of *Aspergillus niger* or *Aspergillus oryzae,* and the *bar* gene of *Streptomyces hygroscopicus.*

The vectors preferably contain an element(s) that permits stable integration of the vector into the genome of the *Fusarium venenatum* host strain or autonomous replication of the vector in the *Fusarium venenatum* host strain independent of the genome of the microorganism.

"Introduction" means introducing a vector comprising the nucleic acid sequence into a *Fusarium venenatum* host strain so that the vector is maintained as a chromosomal integrant or as a self-replicating extra-chromosomal vector. Integration is generally considered to be an advantage as the nucleic acid sequence is more likely to be stably maintained in the *Fusarium venenatum* host strain. Integration of the vector into the chromosome occurs by homologous recombination, non-homologous recombination, or transposition.

The introduction of an expression vector into a *Fusarium venenatum* host strain may involve a process consisting of protoplast formation, transformation of the protoplasts, and regeneration of the cell wall in a manner known *per se.* Suitable methods of transforming *Fusarium* species are described by Malardier et al., 1989, Gene 78: 147-156, and WO 96/00787.

For integration into the genome of a *Fusarium venenatum* host strain, the vector may rely on the nucleic acid sequence encoding the mammalian trypsin or any other element of the vector for stable integration of the vector into the genome by homologous or nonhomologous recombination. Alternatively, the vector may contain additional nucleic acid sequences for directing integration by homologous recombination into the genome of the *Fusarium venenatum* host strain. The additional nucleic acid sequences enable the vector to be integrated into the genome at a precise location(s) in the chromosome(s). To increase the likelihood of integration at a precise location, the integrational elements should preferably contain a sufficient number of nucleic acids, such as 100 to 1,500 base pairs, preferably 400 to 1,500 base pairs, and most preferably 800 to 1,500 base pairs, which are highly homologous with the corresponding target sequence to enhance the probability of homologous recombination. The integrational elements may be any sequences that are homologous with the target sequence in the genome of the *Fusarium venenatum* host strain. Furthermore, the integrational elements may be non-encoding or encoding nucleic acid sequences. On the other hand, the vector may be integrated into the genome of the cell by non-homologous recombination.

For autonomous replication, the vector may further comprise an origin of replication enabling the vector to replicate autonomously in the *Fusarium venenatum* host strain in question. The origin of replication may be any origin of replication mediating autonomous replication which functions in a cell. The term "origin of replication" is defined herein as a sequence that enables a plasmid or vector to replicate independent of chromosomal replication. Examples of a origin of replication useful in a filamentous fungal cell are AMA1 and ANS1 (Gems et al.,1991, Gene 98:61-67; Cullen et al., 1987, Nucleic Acids Research 15: 9163-9175; WO 00/24883). Isolation of the AMA1 gene and construction of plasmids or vectors comprising the gene can be accomplished according to the methods disclosed in WO 00/24883.

The procedures used to ligate the elements described herein to construct the recombinant expression vectors are well known to one skilled in the art (see, *e.g.,* J. Sambrook, E.F. Fritsch, and T. Maniatus, 1989, Molecular Cloning, A Laboratory Manual, 2d edition, Cold Spring Harbor, New York).

In another aspect of the present invention, the *Fusarium venenatum* host strain may contain modifications of one or more genes which encode proteins that may be detrimental to the production, recovery, and/or application of the mammalian trypsin of interest. The modification reduces or eliminates expression of the one or more genes resulting in a mutant cell which may produce more of the mammalian trypsin than the mutant cell without the modification of the gene(s) when cultured under the same conditions.

The gene may encode any protein or enzyme. For example, the enzyme may be an aminopeptidase, amylase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, alpha-glucosidase, beta-glucosidase, invertase, laccase, lipase, mannosidase, mutanase, oxidase, pectinolytic enzyme, peroxidase, phospholipase, phytase, polyphenoloxidase, proteolytic enzyme, ribonuclease, transglutaminase, or xylanase.

### Production and Recovery of the Mammalian Trypsin

The *Fusarium venenatum* host cell is cultivated in a nutrient medium suitable for production of a mammalian trypsin of interest using methods known in the art. For example, the cell may be cultivated by shake flask cultivation, or small-scale or large-scale fermentation (including continuous, batch, fed-batch, or solid state fermentations) in laboratory or industrial fermentors with a suitable medium and under conditions allowing the mammalian trypsin to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g.,* in catalogues of the American Type Culture Collection). The secreted mammalian trypsin can be recovered directly from the medium.

The mammalian trypsin may be detected using methods known in the art that are specific for trypsin. These detection methods may include use of specific antibodies, formation of an enzyme product, disappearance of an enzyme substrate, SDS-PAGE, or any other method known in the art. For example, an enzyme assay may be used to determine the activity of the mammalian trypsin, as described herein.

The resulting mammalian trypsin may be isolated by methods known in the art. For example, the polypeptide may be isolated from the nutrient medium by conventional procedures including, but not limited to, centrifugation, filtration, extraction, spray-drying, evaporation, or precipitation. The isolated mammalian trypsin may then be further purified by a variety of procedures known in the art including, but not limited to, chromatography (*e.g.,* ion exchange, affinity, hydrophobic, chromatofocusing, and size exclusion), electrophoretic procedures (*e.g.,* preparative isoelectric focusing), differential solubility (*e.g.,* ammonium sulfate precipitation), or extraction (see, *e.g.,* Protein Purification, J.-C. Janson and Lars Ryden, editors, VCH Publishers, New York, 1989).

### Uses

The mammalian trypsins produced according to the methods of the present invention may be used in a number of industries including the detergent, leather, chemical, agricultural, pharmaceutical, food, and dairy industries. For example, the mammalian trypsins may be used as a component of a detergent composition as described, for example, in U.S. Patent Nos. 5,288,627, 5,693,520 and 5,948,746. The mammalian trypsins may also be used in numerous applications in the food industry as described, for example, in Owen R. Fennema, ed., in Food *Chemistry,* Marcel Dekker, Inc., New York, 1985. The mammalian trypsins may also be used as a bating enzyme in the leather industry. The mammalian trypsins may be further used in cheese making as described, for example, in U.S. Patent No. 5,948,746.

The present invention is further described by the following examples which should not be construed as limiting the scope of the invention.

### Examples

### Strains and Plasmids

*Fusarium venenatum* host, MLY-3, a morphological mutant of *Fusarium venenatum* strain A3/5 (Royer et al., 1999, Fungal Genet. Biol. 28: 68-78), was used as the recipient strain for the transformation experiments. *Fusarium venenatum* A3/5 was originally deposited as *Fusarium graminearum* ATCC 20334 and recently reclassified as *Fusarium venenatum* by Yoder and Christianson, 1998, Fungal Genetics and Biology 23: 62-80 and O'Donnell et al., 1998, Fungal Genetics and Biology 23: 57-67.

### Nucleotide Sequencing

DNA sequencing was performed on an Perkin-Elmer Biosystems Model 377 XL automated DNA sequencer using dye-terminator chemistry.

### Media and Solutions

VNO3RLMT was composed per liter of 20 ml of 50X Vogels-24mM NaNO₃, 273.33 g of sucrose, and 15 g of LMT Agarose.

50X Vogel's was composed per liter of 125 g of sodium citrate, 250 g of KH₂PO₄, 106.25 g of NaNO₃, 10 g of MgSO₄·7H₂O, 5 g of CaCl₂·2H₂O, 2.5 ml of biotin stock solution (5 mg of biotin in 100 ml of 50% ethanol), and 5 ml of Vogels trace element solution.

Vogels Trace element solution was composed per liter of 50 g of citric acid, 50 g of ZnSO₄·7H₂O (or 2.4 g ZnCl₂), 10 g of Fe(NH₄)₂(SO₄)₂·6H₂O (or 0.68g FeCl₃), 2.5 g of CuSO₄5H₂O, 0.5 g of MnSO₄·H₂O, 0.5 g of H₃BO₃, and 0.5 g of Na₂MoO₄·2H₂O (or (NH₄)₂MoO₄).

BASTA top agarose was composed of COVE top agarose supplemented with 10 mg/ml of the herbicide Basta™ (Hoechst Schering, Rodovre, Denmark).

RA sporulation medium was composed per liter of 50 g of succinic acid, 12.1 g of NaNO₃, 1g of glucose, 20 ml of 50X Vogels, and 0.5 ml of a 10 mg/ml NaMoO₄ stock solution, pH to 6.0.

YEPG medium was composed per liter of 10 g of yeast extract, 20 g of peptone, and 20 g of glucose.

STC was composed of 0.8 M sorbitol, 25 mM Tris pH 8, 25 mM CaCl₂.

SPTC was composed of 40% PEG 4000, 0.8 M sorbitol, 25 mM Tris pH 8, 25 mM CaCl₂.

M400 medium was composed per liter of 50 g of maltodextrin, 2 g of MgSO₄·7H₂O, 2 g of KH₂PO₄, 4 g of citric acid, 8 g of yeast extract, 2 g of urea, 0.5 g of CaCl₂, and 0.5 ml of AMG trace metals solution.

AMG trace metals solution was composed per liter of 14.3 g of ZnSO₄·7H₂O, 2.5 g of CuSO_{4·}5H₂O, 0.5 g of NiCl₂, 13.8 g of FeSO₄, 8.5 g of MnSO₄, and 3.0 g of citric acid.

### Example 1: Construction of pCaHj522

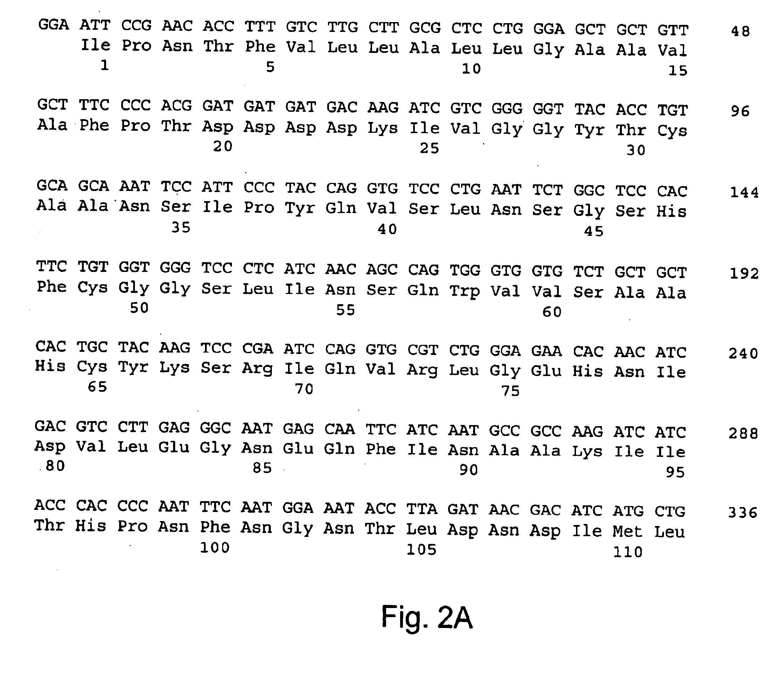

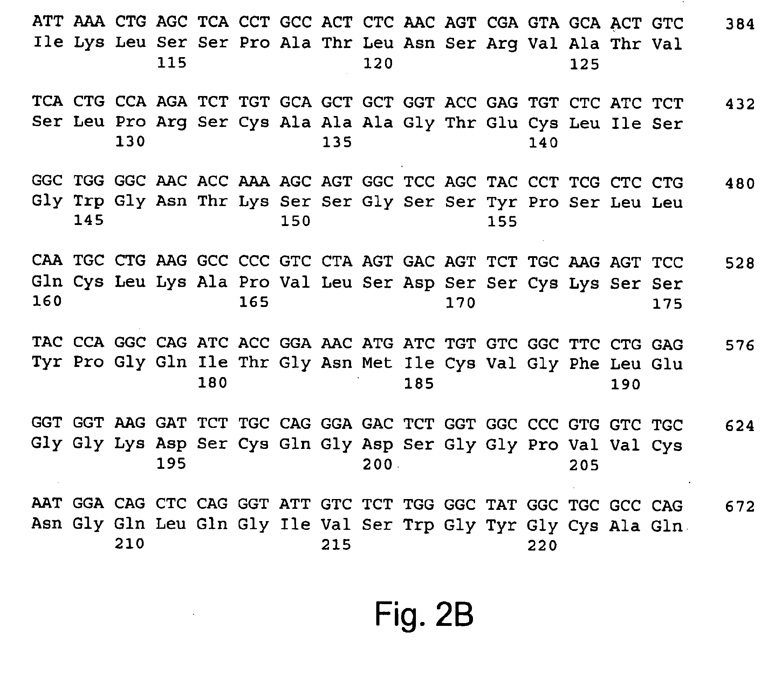

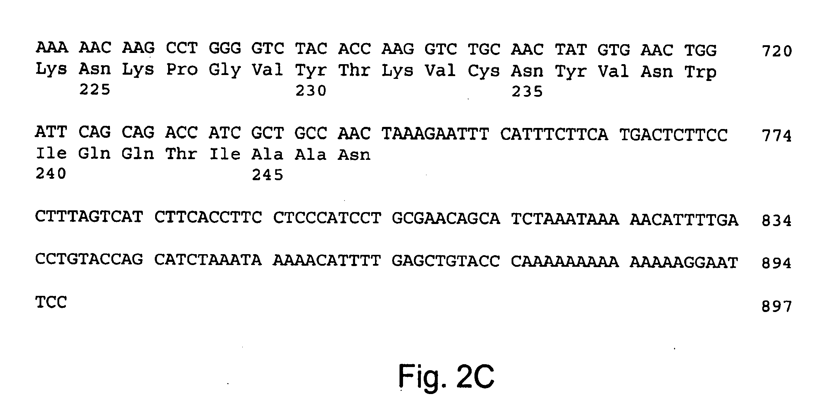

The DNA sequence and deduced amino acid sequence for porcine trypsinogen is shown in Figure 2 (SEQ ID NOs: 3 and 4, respectively). The porcine trypsinogen gene was cloned as a cDNA gene on plasmid p185 as described in WO 97/00316. This cDNA gene was fused in frame to the fragment of the TAKA amylase gene encoding the TAKA amylase signal sequence and then cloned into an *Aspergillus oryzae* expression plasmid. The fragment of the TAKA amylase gene encoding the TAKA amylase signal sequence was PCR amplified from the plasmid TAKA17 that has been described in EP 0 238 023. The *Aspergillus* expression vector used was pCaHj483 described in WO 98/00529. The fusion of the gene fragment encoding the TAKA amylase signal sequence and the fragment encoding the porcine pro trypsin was done using splicing by overlap extension (Horton et al., 1989, Gene 77: 61-68). Pwo DNA polymerase (Roche Molecular Biochemicals, Basel, Switzerland) was used for the PCR amplifications following the manufacturer's instructions. The primers used to PCR amplify the fragment encoding the TAKA amylase signal sequence using a pTAKA17 plasmid preparation as DNA template were:
120464: 5'-TTGGATCCTTTATGATGGTCGCGTGG-3' (SEQ **ID** NO: 5)
120462: 5'-ATCCGTGGGGAAAGCCAAAGCAGGTGCCG-3' (SEQ ID NO: 6)
The primers used to PCR amplify the fragment encoding the porcine pro trypsin using p185 plasmid as DNA template were:
120461: 5'-CCTGCTTTGGCTTTCCCCACGGATGATGAT-3' (SEQ ID NO: 7)
120463: 5'-TTCTCGAGTTAGTTGGCAGCGATGGT-3' (SEQ ID NO: 8)

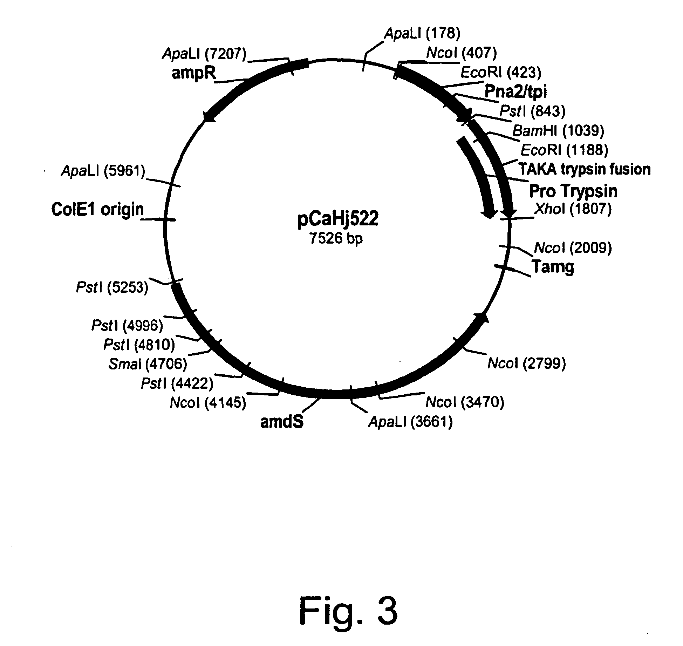

The two PCR reactions were applied to a 1% agarose gel and subjected to agarose gel electrophoresis and the two PCR products were recovered from the gel. The PCR products were mixed and used as template in a third PCR reaction using primers 120464 and 120463. The formed PCR fragment was gel purified in the same way as the two other PCR fragments. The fragment was digested with the restriction enzymes *BamH*I and *Xho*I and inserted into pCaHj483 digested with the same enzymes to form pCaHj522 (Figure 3).

### Example 2: Construction of expression vector pRaMB58

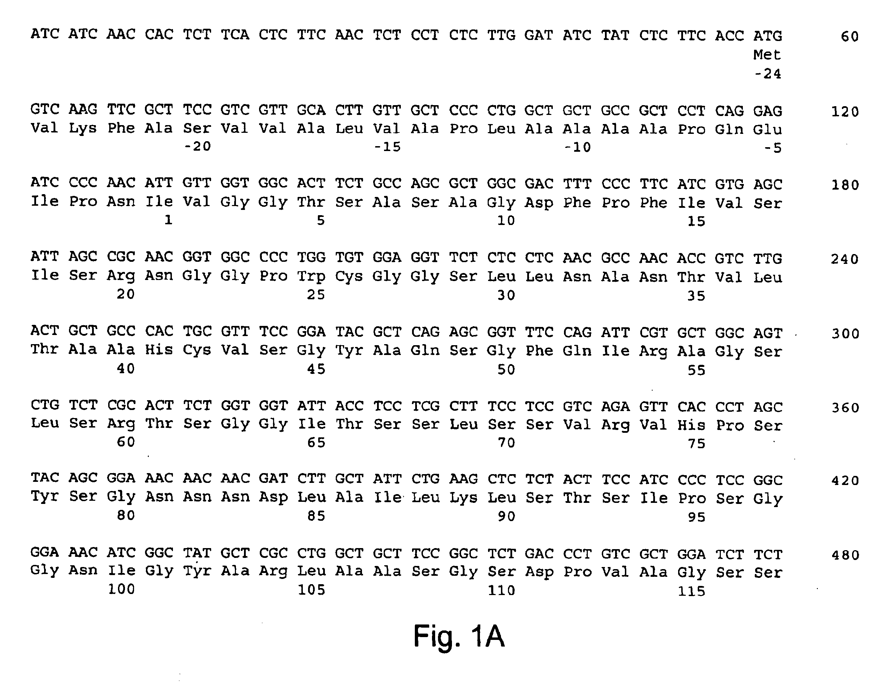

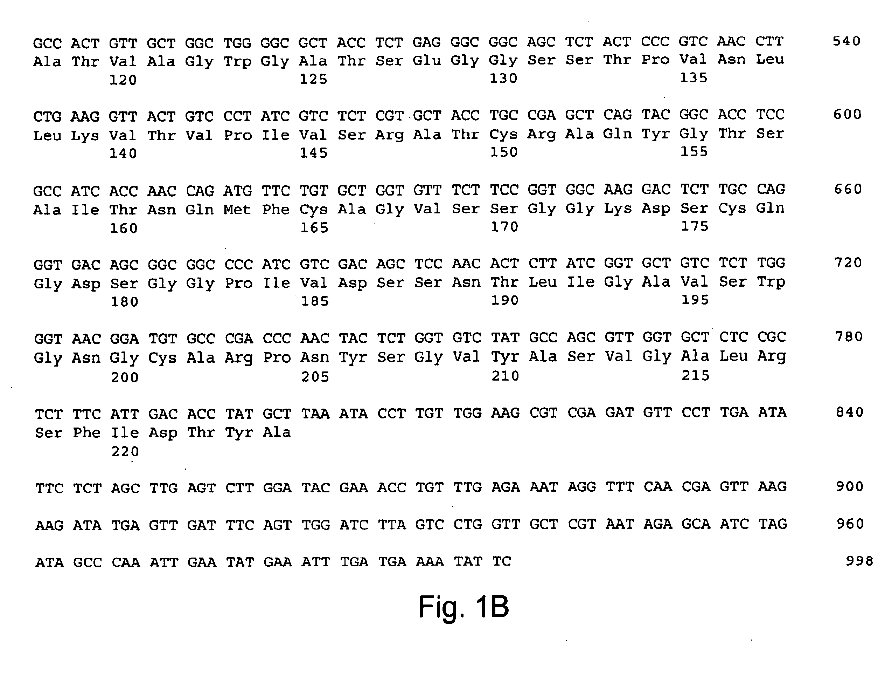

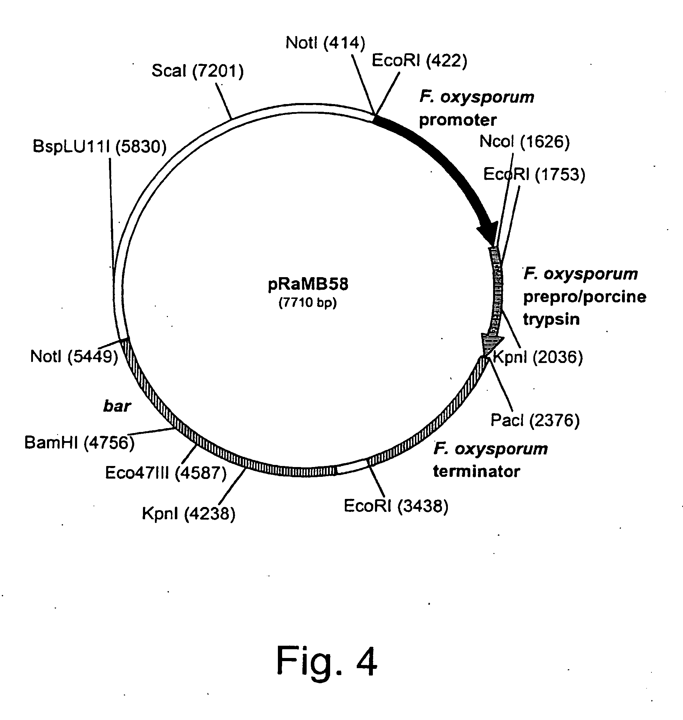

Plasmid pRaMB58 (Figure 4) was constructed for expression of porcine trypsin in *Fusarium venenatum.* First, a DNA segment encoding the signal peptide and propeptide codons of a *Fusarium oxysporum* trypsinogen-like protein (Figure 1; nucleotides 58 to 129 of SEQ ID NO: 1) was amplified using pJRoy6 (U.S. Patent No. 5,837,847) as a template with the following PCR primers:
Primer 980173: 5'-CTTCACCATGGTCAAGTTCGCT-3' (SEQ ID NO: 9)
Primer 980174: 5'-GTTGGGGATCTCCTGAGGAGCG-3' (SEQ ID NO: 10)
   Second, a cDNA segment encoding the mature porcine trypsin shown in Figure 2 (nucleotides 75 to 744 of SEQ ID NO: 3) was amplified using pCaHj522 with the following PCR primers:
Primer 980175: 5'-ATCGTCGGGGGTTACACCTGT-3' (SEQ ID NO: 11)
Primer 980176: 5'-CGTTAATTAATTCTTTAGTTGGCAGCGATGGTCTG-3' (SEQ ID NO: 12)

All PCR reactions utilized high fidelity *Pwo* DNA polymerase and were conducted according to the instructions of the manufacturer (Roche Molecular Biochemicals, Basel, Switzerland). The first PCR fragment was digested with *Nco*I, and the second PCR product was digested with *Pac*l. Both digested fragments were isolated by preparative agarose gel electrophoresis using a 1% agarose gel in 50 mM Tris-base-50 mM borate-1 mM Na₂-EDTA·2H₂O (TBE) buffer and purified using a Bio-Rad Prep-a-Gene Kit according to the manufacturer's instructions (Bio-Rad, Hercules, CA).

The purified fragments were combined in a three-part ligation with pSheB1 (U.S. Patent No. 6,361,973) that was digested with *Nco*l and *Pac*l. The ligation mixture was used to transform *E*. *coli* TOP10 cells (Invitrogen, San Diego, CA) according to the manufacturer's instructions and plasmid DNA from the transformants was digested with *Nco*l plus *Pac*l and screened for the presence of the 0.75 kb trypsin coding sequence using 1% agarose gels in TBE buffer. The resulting vector, designated pRaMB58 (Figure 4), contained a hybrid coding region comprising the *Fusarium oxysporum* trypsin signal peptide and propeptide region followed by the mature porcine trypsin sequence.

### Example 3: Transformation of Fusarium venenatum with pRaMB58

*Fusarium venenatum* MLY-3 (Δtri5) was obtained as described in U.S. Patent No. 6,180,366. Spores of *Fusarium venenatum* MLY-3 were generated by inoculating a flask containing 500 ml of RA sporulation medium with 10 plugs from a 1X Vogels medium plate (2.5% Noble agar) supplemented with 2.5% glucose and 2.5 mM sodium nitrate and incubating at 28°C, 150 rpm for 2 to 3 days. Spores were harvested through MIRACLOTH™ (Calbiochem, San Diego, CA) and centrifuged for 20 minutes at 7000 rpm in a Sorvall RC-5B centrifuge (E. I. DuPont De Nemours and Co., Wilmington, DE). Pelleted spores were washed twice with sterile distilled water, resuspended in a small volume of water, and then counted using a hemocytometer.

Protoplasts were prepared by inoculating 100 ml of YEPG medium with 4 X 10⁷ spores of *Fusarium venenatum* MLY-3 and incubating for 16 hours at 24°C and 150 rpm. The culture was centrifuged for 7 minutes at 3500 rpm in a Sorvall RT 6000D (E. I. DuPont De Nemours and Co., Wilmington, DE). Pellets were washed twice with 30 ml of 1 M MgSO₄ and resuspended in 15 ml of 5 mg/ml of NOVOZYME 234™ (batch PPM 4356, Novozymes A/S, Bagsværd, Denmark) in 1 M MgSO₄. Cultures were incubated at 24°C and 150 rpm until protoplasts formed. A volume of 35 ml of 2 M sorbitol was added to the protoplast digest and the mixture was centrifuged at 2500 rpm for 10 minutes. The pellet was resuspended, washed twice with STC, and centrifuged at 2000 rpm for 10 minutes to pellet the protoplasts. Protoplasts were counted with a hemocytometer and resuspended in an 8:2:0.1 solution of STC:SPTC:DMSO to a final concentration of 1.25 x 10⁷ protoplasts/ml. The protoplasts were stored at -80°C, after controlled-rate freezing in a Nalgene Cryo 1°C Freezing Container (VWR Scientific, Inc., San Francisco, CA).

Frozen protoplasts of *Fusarium venenatum* MLY-3 were thawed on ice. A 100 µg quantity of pRaMB58 was added to a 50 ml sterile polypropylene tube. Two ml of protoplasts were added to the tube, mixed gently, and incubated on ice for 30 minutes. Then 220 µl of SPTC was added and incubated 10 minutes at room temperature followed by 20 ml of SPTC and 10 minutes of further incubation at room temperature. After addition to 500 ml of 40°C VNO3RLMT top agarose, the mixture was poured onto an empty 150 mm diameter plate and incubated overnight at room temperature. Approximately 24 hours later, an additional 25 ml of 40°C VNO3RLMT top agarose containing 10 mg of BASTA™ per ml was poured on top of the plate and incubated at room temperature for up to 14 days. The active ingredient in the herbicide BASTA™ is phosphinothricin. BASTA™ was obtained from AgrEvo (Hoechst Schering, Rodovre, Denmark) and was extracted twice with phenol:chloroform:isoamyl alcohol (25:24:1), and once with chloroform:isoamyl alcohol (24:1) before use.

Four *Fusarium venenatum* transformants were obtained with pRaMB58. The transformants were picked directly from the selection plates (VNO3RLMT underlay with VNO3RLMT-BASTA™ overlay) into 125 ml shake flasks containing 25 ml of M400 medium and incubated at 28°C, 200 rpm on a platform shaker for 6 days. The untransformed recipient strain was also included as a negative control.

One ml aliquots of culture supernatants were harvested at four, five and six days post inoculum. The cell-free supernatants from each transformant were assayed for trypsin activity using N-alpha-benzoyl-DL-arginine p-nitroanilide as substrate using a microtiter plate assay. Specifically, N-alpha-benzoyl-DL-arginine p-nitroanilide was dissolved in DMSO at a concentration of 100 mg/ml and further diluted 1:50 in 100 mM MOPS buffer, 4 mM CaCl₂, 0.01% Triton X-100, pH 7.5 (assay buffer) to a 2 mg/ml solution. The rate of hydrolysis was measured kinetically at 405 nm and 30°C for 3 minutes using a Molecular Devices 96-well plate reader (Molecular Devices, Sunnyvale, CA). Transformants 58c.1, 58c.2, 58c.3, and 58c.4 were found to produce significant trypsin activity.

Table 1 shows that trypsin activity was detected in cultures derived from pRaMB58 transformants, but activity was not detected in an untransformed control strain. The highest levels of trypsin activity were observed after five days, and the yields ranged from approximately 130 to 200 units (per ml). At six days, trypsin activity appeared to decline, suggesting inactivation of the enzyme in older cultures.

**Table 1. Trypsin expression in shake flask cultures of Fusarium venenatum transformants.**

| **Strain/Transformant** | **Trypsin Activity (Units)^{a} after 4 days** | **Trypsin Activity (Units)^{a} after 5 days** | **Trypsin Activity (Units)^{a} after 6 days** |
|---|---|---|---|
| Untransformed control | <10 | <10 | <10 |
| Transformant 58c.1 | 140 | 182 | 74 |
| Transformant 58c.2 | 121 | 140 | 77 |
| Transformant 58c.3 | 83 | 130 | 128 |
| Transformant 58c.4 | 159 | 203 | 139 |

| | | | |
|---|---|---|---|
| ^{a}One trypsin unit was defined as the hydrolysis of one micromole of L-BAPNA per minute per ml of culture broth. | | | |

The broth samples (15 µl) from transformants 58c.1, 58c.2, 58c.3, and 58c.4 were also analyzed by SDS-PAGE using a Novex XCell II mini apparatus (Invitrogen, San Diego, CA). A 15 µl volume of each supernatant sample was heated to 95°C for 5 minutes with an equal volume of Tris-glycine sample buffer (Invitrogen, San Diego, CA). The denatured supernatant proteins were separated on a 10-20% Tris-glycine gradient gel (Invitrogen, San Diego, CA) and stained with Coomassie blue. SDS-PAGE analysis showed that transformants 58c.1, 58c.2, 58c.3, and 58c.4 secrete a prominent polypeptide with an apparent molecular weight of approximately 23 kDa (the expected size of porcine trypsin).

### Example 4: Amino terminal sequencing of trypsin produced by Fusarium venenatum

The denatured supernatant proteins were separated on a 10-20% Tricine SDS-PAGE gradient gel (Invitrogen, San Diego, CA) and electroblotted to onto Novex sequencing grade PVDF membrane (Novex, San Diego, CA) using 10mM CAPS (3-[cyclohexylamino]-1-propanesulfonic acid) in 10% methanol, pH=11.0 for 2 hours at 25Volts. PVDF membrane was stained with 0.1 % Commassie Blue R-250 in 40% MeOH/1% acetic acid for 20 seconds and destained in 50% MeOH to observe the protein bands. SDS-PAGE analysis showed that transformants 58c.1, 58c.2, 58c.3, and 58c.4 secrete a prominent polypeptide with an apparent molecular weight of approximately 23 kDa (the expected size of porcine trypsin).

The 23 kDa SDS-PAGE band from *Fusarium venenatum* transformant 58c.4 (Example 3) was excised and analyzed by N-terminal sequencing. N-Terminal amino acid sequencing of the excised protein was performed on an Applied Biosystems 476A Protein Sequencer (Applied Biosystems, Foster City, CA) with on-line HPLC and liquid phase trifluoroacetic acid (TFA) delivery. Detection of phenylthiohydantoin-amino acids was accomplished by on-line HPLC using Buffer A containing 3.5% tetrahydrofuran in water with 18 ml of the Premix concentrate (Applied Biosystems, Foster City, CA) containing acetic acid, sodium acetate, and sodium hexanesulfonate and Buffer B containing acetonitrile. Data was collected and analyzed with a Macintosh Ilsi using Applied Biosystems 610 Data Analysis software. Sequence determinations were made by visualizing chromatograms against a light source.

The N-terminal sequence determined from this band was IVGGYTXAAN (amino acids 1 to 10 of SEQ ID NO: 4, corresponding to the correct amino acid sequence of mature porcine trypsin. The first four amino acids of mature trypsin, IVGG, are identical to the first four residues in the mature *Fusarium oxysporum* trypsin-like enzyme (amino acids 25 to 28 of SEQ ID NO: 2).

## Claims

1. A method for producing a mammalian trypsin, the method comprising
(a) cultivating a *Fusarium venenatum* host strain in a culture medium under conditions suitable for expression of the mammalian trypsin and secretion thereof into the medium, wherein the *Fusarium venenatum* host strain comprises a nucleic acid construct comprising a nucleic acid sequence encoding the mature coding sequence of a mammalian trypsin operably linked to nucleotides 58 to 129 of SEQ ID NO: 1 encoding the signal peptide and propeptide of *Fusarium oxysporum* trypsinogen; and
(b) recovering the mammalian trypsin from the medium.

2. The method of claim 1, wherein the *Fusarium venenatum* host strain is *Fusarium venenatum* ATCC 20334.

3. The method of claim 1, wherein the *Fusarium venenatum* host strain is a trichothecene-deficient *Fusarium venenatum* strain.

4. The method of claim 1, wherein the *Fusarium venenatum* host strain is a cyclohexadepsipeptide-deficient *Fusarium venenatum* strain.

5. The method of claim 1, wherein the nucleic acid construct further comprises a promoter obtained from a gene selected from the group consisting of an *Aspergillus oryzae* TAKA amylase, *Rhizomucor miehei* aspartic proteinase, *Aspergillus niger* neutral alpha-amylase, *Aspergillus niger* acid stable alpha-amylase, *Aspergillus niger* or *Aspergillus awamori* glucoamylase *(glaA), Rhizomucor miehei* lipase, *Aspergillus oryzae* alkaline protease, *Aspergillus oryzae* triose phosphate isomerase, *Aspergillus nidulans* acetamidase, *Aspergillus oryzae* acetamidase, *Fusarium oxysporum* trypsin-like protease *Fusarium venenatum* AMG, *Fusarium venenatum* Daria, and *Fusarium venenatum* Quinn gene.

6. The method of claim 1, wherein the nucleic acid construct further comprises a promoter obtained from a *Fusarium oxysporum* trypsin-like protease gene.

7. The method of claim 1, wherein the nucleic acid construct further comprises a terminator obtained from a gene selected from the group consisting of an *Aspergillus oryzae* TAKA amylase, *Aspergillus niger* glucoamylase, *Aspergillus nidulans* anthranilate synthase, *Aspergillus niger* alpha-glucosidase, and *Fusarium oxysporum* trypsin-like protease gene.

8. The method of claim 1, wherein the nucleic acid construct further comprises a terminator obtained from a *Fusarium oxysporum* trypsin-like protease gene.

9. The method of any of claims 1 to 8, wherein the mammalian trypsin is a bovine, cow, dog, human, mouse, pig, or rat trypsin.

10. The method of claim 1, wherein the nucleic acid sequence encoding the mature coding sequence of the mammalian trypsin is nucleotides 75 to 744 of SEQ ID NO: 3.

11. The method of claim 1, wherein the mature coding sequence of the mammalian trypsin encodes amino acids 25 to 247 of SEQ ID NO: 4.

12. A nucleic acid construct comprising a nucleic acid sequence encoding the mature coding sequence of a mammalian trypsin operably linked to nucleotides 58 to 129 of SEQ ID NO: 1 encoding the signal peptide and propeptide of *Fusarium oxysporum* trypsinogen.

13. The nucleic acid construct of claim 12, wherein the nucleic acid sequence encoding the mature coding sequence of the mammalian trypsin is nucleotides 75 to 744 of SEQ ID NO: 3.

14. The nucleic acid construct of claim 12, wherein the mature coding sequence of the mammalian trypsin encodes amino acids 25 to 247 SEQ ID NO: 4.

15. A recombinant expression vector comprising the nucleic acid construct of claim 12.

16. A recombinant *Fusarium venenatum* host strain comprising the nucleic acid construct of claim 12.

17. The *Fusarium venenatum* host strain of claim 16, wherein the *Fusarium venenatum* host strain is *Fusarium venenatum* ATCC 20334.

18. The *Fusarium venenatum* host strain of claim 16, wherein the *Fusarium venenatum* host strain is a trichothecene-deficient, a cyclohexadepsipeptide-deficient, or a trichothecene-deficient and a cyclohexadepsipeptide-deficient *Fusarium venenatum* strain.

## Patentansprüche

1. Verfahren zum Herstellen eines Säugertrypsins, wobei das Verfahren umfasst
(a) Kultivieren eines *Fusarium* venenatum-Wirtsstamms in einem Kulturmedium unter Bedingungen, die zur Expression des Säugertrypsins und Sekretion dessen in das Medium geeignet sind, wobei der *Fusarium* venenatum-Wirtsstamm ein Nukleinsäurekonstrukt umfasst, umfassend eine Nukleinsäuresequenz, die die maturierte kodierende Sequenz eines Säugertrypsins kodiert, die funktionsfähig mit den Nukleotiden 58 bis 129 der SEQ ID NO: 1 verknüpft ist, die das Signalpeptid und Propeptid von *Fusarium* oxysporum-Trypsinogen kodieren; und
(b) Rückgewinnen des Säugertrypsins aus dem Medium.

2. Verfahren nach Anspruch 1, wobei der *Fusarium venenatum-Wirtsstamm Fusarium venenatum* ATCC 20334 ist.

3. Verfahren nach Anspruch 1, wobei der *Fusarium venenatum*-Wirtsstamm ein Trichothecen-defizienter *Fusarium venenatum-Stamm* ist.

4. Verfahren nach Anspruch 1, wobei der *Fusarium* venenatum-Wirtsstamm ein Cyclohexadepsipeptid-defizienter *Fusarium venenatum-Stamm* ist.

5. Verfahren nach Anspruch 1, wobei das Nukleinsäurekonstrukt weiterhin einen Promotor umfasst, erhalten aus einem Gen, ausgewählt aus der Gruppe bestehend aus einem *Aspergillus oryzae* TAKA-Amylase-, *Rhizomucor miehei* Aspartatproteinase-, *Aspergillus niger* neutrale alpha-Amylase-, *Aspergillus niger* säurestabile alpha-Amylase-, *Aspergillus niger* oder *Aspergillus awamori* Glucoamylase *(glaA)-, Rhizomucor miehei* Lipase-, *Aspergillus oryzae* alkalische Protease-, *Aspergillus oryzae* Triosephosphatisomerase-, *Aspergillus nidulans* Acetamidase-, *Aspergillus oryzae* Acetamidase-, *Fusarium oxysporum* Trypsin-artiges Protease-, *Fusarium venenatum* AMG-, *Fusarium venenatum* Daria- und *Fusarium venenatum* Quinn- Gen.

6. Verfahren nach Anspruch 1, wobei das Nukleinsäurekonstrukt weiterhin einen Promotor umfasst, erhalten aus einem *Fusarium oxysporum* Trypsin-artigem Protease Gen.

7. Verfahren nach Anspruch 1, wobei das Nukleinsäurekonstrukt weiterhin einen Terminator umfasst, erhalten aus einem Gen, ausgewählt aus der Gruppe bestehend aus *Aspergillus oryzae* TAKA-Amylase, *Aspergillus niger* Glucoamylase, *Aspergillus nidulans* Anthranilatsynthase, *Aspergillus niger* alpha-Glucosidase und *Fusarium oxysporum* Trypsin-artigem Protease Gen.

8. Verfahren nach Anspruch 1, wobei das Nukleinsäurekonstrukt weiterhin einen Terminator umfasst, erhalten aus einem *Fusarium oxysporum*-Trypsin-artigem Protease Gen.

9. Verfahren nach einem beliebigen der Ansprüche 1-8, wobei das Säugertrypsin ein Rinder- , Kuh-, Hunde-, Human-, Maus-, Schweine- oder Rattentrypsin ist.

10. Verfahren nach Anspruch 1, wobei die Nukleinsäuresequenz, die die maturierte kodierende Sequenz des Säugertrypsins kodiert, Nukleotide 75 bis 744 der SEQ ID NO: 3 ist.

11. Verfahren nach Anspruch 1, wobei die maturierte kodierende Sequenz des Säugertrypsins Aminosäuren 25 bis 247 der SEQ ID NO: 4 kodiert.

12. Nukleinsäurekonstrukt, umfassend eine Nukleinsäuresequenz, die die maturierte kodierende Sequenz eines Säugertrypsins kodiert, funktionsfähig verknüpft zu Nukleotiden 58 bis 129 der SEQ ID NO: 1, die das Signalpeptid und Propeptid von *Fusarium oxysporum*-Trypsinogen kodieren.

13. Nukleinsäurekonstrukt nach Anspruch 12, wobei die Nukleinsäuresequenz, die die maturierte kodierende Sequenz des Säugertrypsins kodiert, Nukleotide 75 bis 744 der SEQ ID NO: 3 ist.

14. Nukleinsäurekonstrukt nach Anspruch 12, wobei die maturierte kodierende Sequenz des Säugertrypsins Aminosäuren 25 bis 247 der SEQ ID NO: 4 kodiert.

15. Rekombinanter Expressionsvektor, umfassend das Nukleinsäurekonstrukt gemäß Anspruch 12.

16. Rekombinanter *Fusarium venenatum*-Wirtsstamm, umfassend das Nukleinsäurekonstrukt gemäß Anspruch 12.

17. *Fusarium* venenatum-Wirtsstamm nach Anspruch 16, wobei der *Fusarium venenatum-*Wirtsstamm *Fusarium venenatum-*ATCC 20334 ist.

18. *Fusarium venenatum*-Wirtsstamm nach Anspruch 16, wobei der *Fusarium venenatum-*Wirtsstamm ein Trichothecen-defizienter, ein Cyclohexadepsipeptid-defizienter, oder ein Trichothecen-defizienter und ein Cyclohexadepsipeptid-defizienter *Fusarium venenatum-*Stamm ist.

## Revendications

1. Méthode pour produire une trypsine de mammifère, la méthode comprenant
(a) cultiver une souche hôte *Fusarium venenatum* dans un milieu de culture dans des conditions appropriées pour l'expression de la trysine de mammifère et la sécrétion de celle-ci dans le milieu, dans laquelle la souche hôte *Fusarium venenatum* comprend une construction d'acide nucléique comprenant une séquence d'acide nucléique codant pour la séquence codante mature d'une trypsine de mammifère liée de façon opérationnelle aux nucléotides 58 à 129 de la SEQ ID NO : 1 codant pour le peptide signal et le propeptide du trypsinogène de *Fusarium oxysporum ;* et
(b) récupérer la trypsine de mammifère à partir du milieu.

2. Méthode selon la revendication 1, dans laquelle la souche hôte *Fusarium venenatum* est *Fusarium venenatum* ATCC 20334.

3. Méthode selon la revendication 1, dans laquelle la souche hôte *Fusarium venenatum* est une souche de *Fusarium venenatum* déficiente en trichothécène.

4. Méthode selon la revendication 1, dans laquelle la souche hôte *Fusarium venenatum* est une souche de *Fusarium venenatum* déficiente en cyclohexadepsipeptide.

5. Méthode selon la revendication 1, dans laquelle la construction d'acide nucléique comprend en outre un promoteur obtenu à partir d'un gène choisi dans le groupe constitué de l'amylase TAKA *d'Aspergillus oryzae,* de la protéinase aspartique de *Rhizomucor miehei,* de l'alpha-amylase neutre *d'Aspergillus niger,* de l'alpha-amylase acide stable *d'Aspergillus niger,* de la glucoamylase *(glaA) d'Aspergillus niger ou d'Aspergillus awamori,* de la lipase de *Rhizomucor miehei,* de la protéase alcaline *d'Aspergillus oryzae,* de la triose phosphate isomérase *d'Aspergillus oryzae,* de l'acétamidase *d'Aspergillus nidulans,* de l'acétamidase *d'Aspergillus oryzae,* de la protéase ressemblant à la trypsine de *Fusarium oxysporum,* de l'AMG de *Fusarium venenatum,* du gène Daria de *Fusarium venenatum,* et du gène Quinn de *Fusarium venenatum.*

6. Méthode selon la revendication 1, dans laquelle la construction d'acide nucléique comprend en outre un promoteur obtenu à partir d'un gène d'une protéase ressemblant à la trypsine de *Fusarium oxysporum.*

7. Méthode selon la revendication 1, dans laquelle la construction d'acide nucléique comprend en outre un terminateur obtenu à partir d'un gène choisi dans le groupe constitué de l'amylase TAKA *d'Aspergillus oryzae,* de la glucoamylase *d'Aspergillus niger,* de l'anthranilate synthase *d'Aspergillus nidulans,* de l'alpha-glucosidase *d'Aspergillus niger,* et d'un gène de la protéase ressemblant à la trypsine de *Fusarium oxysporum.*

8. Méthode selon la revendication 1, dans laquelle la construction d'acide nucléique comprend en outre un terminateur obtenu à partir d'un gène d'une protéase ressemblant à la trypsine de *Fusarium oxysporum.*

9. Méthode selon l'une quelconque des revendications 1 à 8, dans laquelle la trypsine de mammifère est une trypsine de bovin, de vache, de chien, d'humain, de souris, de cochon ou de rat.

10. Méthode selon la revendication 1, dans laquelle la séquence d'acide nucléique codant pour la séquence codante mature de la trypsine de mammifère est les nucléotides 75 à 744 de la SEQ ID NO :3.

11. Méthode selon la revendication 1, dans laquelle la séquence codante mature de la trypsine de mammifère code les acides aminés 25 à 247 de la SEQ ID NO :4.

12. Construction d'acide nucléique comprenant une séquence d'acide nucléique codant pour la séquence codante mature d'une trypsine de mammifère lié de façon opérationnelle aux nucléotides 58 à 129 de la SEQ ID NO : 1 codant pour le peptide signal et le propeptide du trypsinogène de *Fusarium oxysporum.*

13. Construction d'acide nucléique selon la revendication 12, dans laquelle la séquence d'acide nucléique codant pour la séquence codante mature de la trypsine de mammifère est les nucléotides 75 à 744 de la SEQ ID NO :3.

14. Construction d'acide nucléique selon la revendication 12, dans laquelle la séquence codante mature de la trypsine de mammifère code les acides aminés 25 à 247 de la SEQ ID NO:4.

15. Vecteur d'expression recombinant comprenant une construction d'acide nucléique selon la revendication 12.

16. Souche hôte recombinante de *Fusarium venenatum* comprenant une construction d'acide nucléique selon la revendication 12.

17. Souche hôte de *Fusarium venenatum* selon la revendication 16, dans laquelle la souche hôte de *Fusarium venenatum* est *Fusarium venenatum* ATCC 20334.

18. Souche hôte de *Fusarium venenatum* selon la revendication 16, dans laquelle la souche hôte de *Fusarium venenatum* est une souche de *Fusarium venenatum* décifiente en trichothécène, en cyclohexadepsipeptide, ou en trichothécène et en cyclohexadepsipeptide.


### SEQUENCE LISTING

<110> Novozymes Biotech, Inc.
<120> Methods For Producing Mammalian Trypsins
<130> 10333.204-WO
<150> 60/407170
   <151> 2002-08-30
<160> 12
<170> Patent In version 3.2
<210> 1
   <211> 998
   <212> DNA
   <213> Fusarium oxysporum
<400> 1
   atcatcaacc actcttcact cttcaactct cctctcttgg atatctatct cttcaccatg 60
   gtcaagttcg cttccgtcgt tgcacttgtt gctcccctgg ctgctgccgc tcctcaggag 120
   atccccaaca ttgttggtgg cacttctgcc agcgctggcg actttccctt catcgtgagc 180
   attagccgca acggtggccc ctggtgtgga ggttctctcc tcaacgccaa caccgtcttg 240
   actgctgccc actgcgtttc cggatacgct cagagcggtt tccagattcg tgctggcagt 300
   ctgtctcgca cttctggtgg tattacctcc tcgctttcct ccgtcagagt tcaccctagc 360
   tacagcggaa acaacaacga tcttgctatt ctgaagctct ctacttccat cccctccggc 420
   ggaaacatcg gctatgctcg cctggctgct tccggctctg accctgtcgc tggatcttct 480
   gccactgttg ctggctgggg cgctacctct gagggcggca gctctactcc cgtcaacctt 540
   ctgaaggtta ctgtccctat cgtctctcgt gctacctgcc gagctcagta cggcacctcc 600
   gccatcacca accagatgtt ctgtgctggt gtttcttccg gtggcaagga ctcttgccag 660
   ggtgacagcg gcggccccat cgtcgacagc tccaacactc ttatcggtgc tgtctcttgg 720
   ggtaacggat gtgcccgacc caactactct ggtgtctatg ccagcgttgg tgctctccgc 780
   tctttcattg acacctatgc ttaaatacct tgttggaagc gtcgagatgt tccttgaata 840
   ttctctagct tgagtcttgg atacgaaacc tgtttgagaa ataggtttca acgagttaag 900
   aagatatgag ttgatttcag ttggatctta gtcctggttg ctcgtaatag agcaatctag 960
   atagcccaaa ttgaatatga aatttgatga aaatattc 998
<210> 2
   <211> 248
   <212> PRT
   <213> Fusarium oxysporum
<400> 2
   Met Val Lys Phe Ala Ser Val Val Ala Leu Val Ala Pro Leu Ala Ala
   1 5 10 15
   Ala Ala Pro Gln Glu Ile Pro Asn Ile Val Gly Gly Thr Ser Ala Ser
   20 25 30
   Ala Gly Asp Phe Pro Phe Ile Val Ser Ile Ser Arg Asn Gly Gly Pro
   35 40 45
   Trp Cys Gly Gly Ser Leu Leu Asn Ala Asn Thr Val Leu Thr Ala Ala
   50 55 60
   His Cys Val Ser Gly Tyr Ala Gln Ser Gly Phe Gln Ile Arg Ala Gly
   65 70 75 80
   Ser Leu Ser Arg Thr Ser Gly Gly Ile Thr Ser Ser Leu Ser Ser Val
   85 90 95
   Arg Val His Pro Ser Tyr Ser Gly Asn Asn Asn Asp Leu Ala Ile Leu
   100 105 110
   Lys Leu Ser Thr Ser Ile Pro Ser Gly Gly Asn Ile Gly Tyr Ala Arg
   115 120 125
   Leu Ala Ala Ser Gly Ser Asp Pro Val Ala Gly Ser Ser Ala Thr Val
   130 135 140
   Ala Gly Trp Gly Ala Thr Ser Glu Gly Gly Ser Ser Thr Pro Val Asn
   145 150 155 160
   Leu Leu Lys Val Thr Val Pro Ile Val Ser Arg Ala Thr Cys Arg Ala
   165 170 175
   Gln Tyr Gly Thr Ser Ala Ile Thr Asn Gln Met Phe Cys Ala Gly Val
   180 185 190
   Ser Ser Gly Gly Lys Asp Ser Cys Gln Gly Asp Ser Gly Gly Pro Ile
   195 200 205
   Val Asp Ser Ser Asn Thr Leu Ile Gly Ala Val Ser Trp Gly Asn Gly
   210 215 220
   Cys Ala Arg Pro Asn Tyr Ser Gly Val Tyr Ala Ser Val Gly Ala Leu
   225 230 235 240
   Arg Ser Phe Ile Asp Thr Tyr Ala
   245
<210> 3
   <211> 897
   <212> DNA
   <213> Porcine
<400> 3
   ggaattccga acacctttgt cttgcttgcg ctcctgggag ctgctgttgc tttccccacg      60
   gatgatgatg acaagatcgt cgggggttac acctgtgcag caaattccat tccctaccag    120
   gtgtccctga attctggctc ccacttctgt ggtgggtccc tcatcaacag ccagtgggtg    180
   gtgtctgctg ctcactgcta caagtcccga atccaggtgc gtctgggaga acacaacatc    240
   gacgtccttg agggcaatga gcaattcatc aatgccgcca agatcatcac ccaccccaat    300
   ttcaatggaa ataccttaga taacgacatc atgctgatta aactgagctc acctgccact    360
   ctcaacagtc gagtagcaac tgtctcactg ccaagatctt gtgcagctgc tggtaccgag    420
   tgtctcatct ctggctgggg caacaccaaa agcagtggct ccagctaccc ttcgctcctg    480
   caatgcctga aggcccccgt cctaagtgac agttcttgca agagttccta cccaggccag    540
   atcaccggaa acatgatctg tgtcggcttc ctggagggtg gtaaggattc ttgccaggga    600
   gactctggtg gccccgtggt ctgcaatgga cagctccagg gtattgtctc ttggggctat    660
   ggctgcgccc agaaaaacaa gcctggggtc tacaccaagg tctgcaacta tgtgaactgg    720
   attcagcaga ccatcgctgc caactaaaga atttcatttc ttcatgactc ttccctttag    780
   tcatcttcac cttcctccca tcctgcgaac agcatctaaa taaaaacatt ttgacctgta    840
   ccagcatcta aataaaaaca ttttgagctg tacccaaaaa aaaaaaaaag gaattcc        897
<210> 4
   <211> 247
   <212> PRT
   <213> Porcine
<400> 4
   Ile Pro Asn Thr Phe Val Leu Leu Ala Leu Leu Gly Ala Ala Val Ala
   1 5 10 15
   Phe Pro Thr Asp Asp Asp Asp Lys Ile Val Gly Gly Tyr Thr Cys Ala
   20 25 30
   Ala Asn Ser Ile Pro Tyr Gln Val Ser Leu Asn Ser Gly Ser His Phe
               35                  40                  45
   Cys Gly Gly Ser Leu Ile Asn Ser Gln Trp Val Val Ser Ala Ala His
       50                  55                  60
   Cys Tyr Lys Ser Arg Ile Gln Val Arg Leu Gly Glu His Asn Ile Asp
   65                  70                  75                  80
   Val Leu Glu Gly Asn Glu Gln Phe Ile Asn Ala Ala Lys Ile Ile Thr
                   85                  90                  95
   His Pro Asn Phe Asn Gly Asn Thr Leu Asp Asn Asp Ile Met Leu Ile
               100                 105                 110
   Lys Leu Ser Ser Pro Ala Thr Leu Asn Ser Arg Val Ala Thr Val Ser
           115                 120                 125
   Leu Pro Arg Ser Cys Ala Ala Ala Gly Thr Glu Cys Leu Ile Ser Gly
       130                 135                 140
   Trp Gly Asn Thr Lys Ser Ser Gly Ser Ser Tyr Pro Ser Leu Leu Gln
   145                 150                 155                 160
   Cys Leu Lys Ala Pro Val Leu Ser Asp Ser Ser Cys Lys Ser Ser Tyr
                   165                 170                 175
   Pro Gly Gln Ile Thr Gly Asn Met Ile Cys Val Gly Phe Leu Glu Gly
               180                 185                 190
   Gly Lys Asp Ser Cys Gln Gly Asp Ser Gly Gly Pro Val Val Cys Asn
           195                 200                 205
   Gly Gln Leu Gln Gly Ile Val Ser Trp Gly Tyr Gly Cys Ala Gln Lys
       210                 215                 220
   Asn Lys Pro Gly Val Tyr Thr Lys Val Cys Asn Tyr Val Asn Trp Ile
   225                 230                 235                 240
   Gln Gln Thr Ile Ala Ala Asn
                   245
<210> 5
   <211> 26
   <212> DNA
   <213> Aspergillus oryzae
<400> 5
   ttggatcctt tatgatggtc gcgtgg 26
<210> 6
   <211> 29
   <212> DNA
   <213> Aspergillus oryzae
<400> 6
   atccgtgggg aaagccaaag caggtgccg 29
<210> 7
   <211> 30
   <212> DNA
   <213> Porcine
<400> 7
   cctgctttgg ctttccccac ggatgatgat 30
<210> 8
   <211> 26
   <212> DNA
   <213> Porcine
<400> 8
   ttctcgagtt agttggcagc gatggt 26
<210> 9
   <211> 22
   <212> DNA
   <213> Fusarium oxysporum
<400> 9
   cttcaccatg gtcaagttcg ct 22
<210> 10
   <211> 22
   <212> DNA
   <213> Fusarium oxysporum
<400> 10
   gttggggatc tcctgaggag cg 22
<210> 11
   <211> 21
   <212> DNA
   <213> Porcine
<400> 11
   atcgtcgggg gttacacctg t 21
<210> 12
   <211> 35
<212> DNA
   <213> Porcine
<400> 12
   cgttaattaa ttctttagtt ggcagcgatg gtctg 35